# EUROPEAN PATENT SPECIFICATION

(11) **EP 3 347 715 B1**
(45) Date of publication and mention of the grant of the patent: **26.08.2020**
(21) Application number: 16765953.1
(22) Date of filing: 08.09.2016
(51) Int. Cl.: G01N 33/569, C12Q 1/6883

(54) **METHOD AND KIT FOR DIAGNOSING EPITHELIAL-TO-MESENCHYMAL TRANSITION (EMT) OF THE PERITONEUM**
VERFAHREN UND KIT ZUR DIAGNOSE DES EPITHELIAL-MESENCHYMAL-ÜBERGANGS (EMT) DES PERITONEUMS
PROCÉDÉ ET KIT DE DIAGNOSTIC DE TRANSITION ÉPITHÉLIO-MÉSENCHYMATEUSE (TEM) DU PÉRITOINE

(30) Priority: 09.09.2015 DE 102015115158
(43) Date of publication of application: 18.07.2018
(73) Proprietor: Fresenius Medical Care Deutschland GmbH, 61352 Bad Homburg (DE)
(72) Inventor: PLASSLICK-DEETJEN, Jutta, 77960 Seelbach (DE); BÜCHEL, Janine, 61350 Bad Homburg (DE); STEPPAN, Sonja, 63263 Neu-Isenburg (DE); LOPEZ-CABRERA, Manuel, 28049 Madrid (ES); AGUILERA, Abelardo, 28006 Madrid (ES); SELGAS, Rafael, 28046 Madrid (ES)
(74) Representative: Dörries, Hans Ulrich
(86) International application number: PCT/EP2016/071149
(87) International publication number: WO 2017/042253

(56) References cited:
- WO-A1-2004/065602
- WO-A1-2010/064702
- US-A1- 2015 190 430
- Vicente Ruiz Carpio: "An?lisis de la huella gen?tica de la transici?n mesotelio mesenquimal: caracterizaci?n de nuevos marcadores en di?lisis peritoneal", TESIS DOCTORAL, 11 March 2016 (2016-03-11), XP055311840, Retrieved from the Internet: URL:http://eprints.ucm.es/36335/1/T36935.p df [retrieved on 2016-10-18]

## Description

The invention relates to the field of diagnosis of epithelial-to-mesenchymal transition (EMT), in particular, mesothelial-to-mesenchymal transition (MMT), in particular EMT of the peritoneum, which often occurs upon peritoneal dialysis. The invention provides a method and a use of a kit based on markers comprising the extracellular matrix proteins collagen 13, collagen 6, and keratin 34, matrix metalloproteinase 1, which is a protein involved in building and/or restructuring of extracellular matrix, cadherin 13 and thrombospondin 1, which are involved in cell-cell and/or cell-matrix contacts, the growth factor VEGF, and the BMP antagonist Gremlin 1.

Peritoneal dialysis (PD) is an effective and cost-efficient alternative to hemodialysis (HD) in the treatment of end stage renal disease, covering about 10-15% of the dialysis population. During PD, the peritoneal membrane or peritoneum functions as a semipermeable membrane across which ultrafiltration and diffusion take place. However, the long-term use of PD is still limited. Nearly 50% of patients are forced to switch to hemodialysis within four or five years of PD treatment, as prolonged exposure to bioincompatible PD fluid, peritonitis and hemoperitoneum episodes induce degenerative changes of the peritoneal tissue that ultimately lead to ultrafiltration failure. These progressive functional and morphological alterations of the peritoneum may be caused by high concentrations of glucose, low pH and lactate buffer in the PD fluid, and are reflected by inflammatory reactions, gradual denudation of the mesothelial cell (MC) monolayer, submesothelial fibrosis and neoangiogenesis.

During long-term PD, MCs undergo a progressive loss of epithelial characteristics and acquire a myofibroblast-like phenotype. This epithelial-to-mesenchymal transition (EMT) is a complex and stepwise process that is characterized by the loss of apical-basolateral polarity, disruption of intercellular junctions and acquisition of migratory and invasive properties (Selgas et al., 2006, Nephrol Dial Transplant 21 [Suppl 2]:ii2-ii7; Aroeira et al. 2007, J Am Soc Nephrol 18:2004-2013). MCs that undergo EMT acquire the ability to produce extracellular matrix components as well as inflammatory, fibrogenic and angiogenic factors. During the course of EMT of the peritoneal membrane, peritoneal fibrosis and angiogenesis are triggered via the upregulation of transforming growth factor TGF-β1 and vascular endothelial growth factor (VEGF).

While, so far, changes of the peritoneum during PD cannot be avoided, optimized treatment, e.g., with biocompatible solutions or another treatment scheme (e.g., changing the fill volume and/or dwell time), can at least delay these changes. EMT is a reversible process, at least in the early stages. Furthermore, the efficiency of the PD treatment may be optimized based on the status of the peritoneum of the patient.

EMT is a biological process which is not only involved in changes to the peritoneum during PD, but which also plays a role in embryogenesis and wound healing. EMT can also cause organ fibrosis and play a role in cancer progression and metastasis. EMT has also shown to contribute to the pathogenesis of various diseases, such as cancer or degenerative fibrotic disorders in different organs, including the lung (WO 2014/139885 A2).

WO 2014/139885 A2 teaches that matrix metalloproteinase MMP10, cellular and soluble fibronectin, E-cadherin and vimentin are markers for EMT. WO 2004/065602 A1 discloses a method for the diagnosis of EMT by detecting a plurality of markers in a sample including integrin α2, snail, integrin α3, cadherin E, cytokeratin, CD9 and CD151. In the prior art, several further markers for EMT or, generally, fibrosis, which is associated with EMT, have been identified. For example, WO2011/054893 A2 shows an association between the level of TLR-9 expression and fibrosis. WO2012/042091 teaches a method of determining the progression of fibrosis based on determination of TAK1. US 2013/0303563 A1 discloses that periostin, HSPG degradation, PDGF, leptin, and CD68+ macrophage density are markers for peritoneal injury. US 2013/020940 A1 suggests that determination of BMP9 or BMP10 is useful for assessing the risk of developing a fibrotic disorder. Aroeira et al., 2007, teach a decrease in expression of E-cadherin, cytokeratins, claudins, occludins, desmoplakin, ZO-1, mucin-1, tPA and CD34, and an increase in expression of N-cadherin, snail, vimentin, TGF-β, fibronectin, extracellular matrix proteins, in particular collagen I and III, αSMA, FGF- 1 and FGF-2, MMP-2 and MMP-9, FSP-1, PAI-1, VEGF, and cyclooxygenase -2 in PD patients' peritoneum.

However, a need to provide markers for EMT, which closely correlate with the function of the tissue undergoing EMT, e.g., the filtration capacity of peritoneum in PD, remains. The inventors have solved the problem of providing such markers, a corresponding use of a kit and a method for diagnosing EMT of the peritoneum. The subject matter of the invention is described, e.g., by the appended claims.

The invention provides a method for the diagnosis of epithelial-to-mesenchymal transition (EMT) of the peritoneum, comprising detecting the absence and/or amount of markers in a sample, wherein the markers are cadherin 13, collagen 13, collagen 6, keratin 34, matrix metalloproteinase 1, thrombospondin 1, VEGF and Gremlin 1, wherein the sample is selected from the group comprising
a) a sample from a patient comprising peritoneal effluent, peritoneal fluid, serum, peritoneal tissue, and
b) culture medium or cell lysate from a cell or tissue culture useful as a model for peritoneal dialysis.

In the context of the present invention, the article "a" is understood to mean "one or more", unless explicitly specified otherwise. Thus, e.g., the markers may also comprise two or three (or more) extracellular marker proteins or two or three proteins involved in cell-cell and/or cell-matrix contacts.

The invention also provides a corresponding use of a kit for the detection of markers in a sample for the diagnosis of epithelial-to-mesenchymal transition (EMT) of the peritoneum, the kit comprising agents for the detection of the markers cadherin 13, collagen 13, collagen 6, keratin 34, matrix metalloproteinase 1, thrombospondin 1, VEGF and Gremlin 1, wherein the sample is selected from the group comprising
a) a sample from a patient comprising peritoneal effluent, peritoneal fluid, serum, peritoneal tissue, and
b) culture medium or cell lysate from a cell or tissue culture useful as a model for peritoneal dialysis,
wherein the agents for the detection of the markers are antibodies or fragments thereof which are suitable for the detection of the markers in protein form, or nucleic acids which are suitable for the detection of the markers in nucleic acid form.

According to one embodiment of the invention, if the markers are in protein form, the agents for the detection of the markers in the sample are antibodies or fragments thereof. Antibodies are suitable for the detection of the markers in protein form. Antibody or fragments thereof may be of human, mouse, rat, rabbit, guinea pig, goat, cat, avian, e.g., chicken, or chimeric origin. They may be polyclonal or monoclonal, or generated by genetic engineering. Antibody fragments comprise, e.g., Fab Fragments, Fab₂ fragments, scFv.

Alternatively, the agents for detection of the markers in the sample may be nucleic acids, e.g., RNA or DNA, which are suitable for detection of the markers in nucleic acid form, in particular, for detection of RNA transcripts of the markers or cDNA derived therefrom. The nucleic acids are preferably at least partly single stranded and are capable of hybridizing under stringent conditions with nucleic acids encoding the markers.

According to another embodiment of the invention, the agents for the detection of the markers in the sample are linked to a solid support, e.g., a chip, beads of different sizes which may be magnetic, etc. In a preferred embodiment, the kit comprises a microarray, in particular, an antibody microarray, also designated antibody chip. Suitable antibody chip formats that can be adapted to the invention are disclosed, e.g., in Wingren et al., 2009, Methods Mol Biol. 509:57-84; Chaga et al., 2008, methods Mol Biol. 441:129-51, or can be custom-made by companies such as Abnova Corporation, R&D Systems, Full Moon BioSystems, Raybiotech, Inc., BioSims and others.

The inventors analyzed the gene expression of healthy mesothelial cells (obtained from the omentum of patients without renal failure), epithelioid cells (i.e., cells in the first steps of EMT of mesothelial cells/MMT, with a phenotype similar to mesothelial cells, obtained from effluents of PD patients), and cells in an advanced stage of EMT of the peritoneum (i.e., cells that have changed their phenotype, also termed non-epithelioid). Several genes were identified that are significantly and specifically upregulated once the cells have acquired the non-epithelioid phenotype. Their expression, and correlation with function, was verified, checking the respective protein levels in different body fluids, including serum and effluent. The inventors surprisingly found that markers from several functional groups play an important role in EMT of the peritoneum. The inventors further found that both RNA and protein levels of these markers are correlated with clinical parameters available from the patients, and their regulation allows for functional conclusions. In particular, the markers of the invention also make it possible to differentiate between early and advanced phases of EMT of the peritoneum. For example, the mRNA levels of CDH13, COL6A3, COL13A1, THBS1 and MMP1 allow for discrimination between an early and advanced phase of the EMT of the peritoneum, and correlate with the clinical data of peritoneal water transport / ultrafiltration.

At least three of the markers analyzed according to the invention are an extracellular matrix protein comprising keratin 34 (KRT34), and the collagens collagen 13 (COL13A1) and collagen 6 (COL6A1). Alternative or additional markers from the group of extracellular matrix proteins comprise collagen 1 and/or 3 (Selgas et al., 2006, Aroeira et al., 2007).

At least one of the markers analyzed according to the invention is a protein involved in building and/or restructuring of extracellular matrix comprising matrix metalloproteinase 1 (MMP1). Alternative or additional markers from the group of proteins involved in building and/or restructuring of extracellular matrix comprise the matrix metalloproteinases MMP2, MMP9 and/or MMP10 (Selgas et al., 2006, Aroeira et al., 2007, US2013/0303563 A1, WO 2014/139885 A2)

At least two of the markers analyzed according to the invention are proteins involved in cell-cell and/or cell-matrix contacts comprising cadherin 13 (CDH13) and thrombospondin 1 (THBS1 or TPS1).

Alternative or additional markers from the group of proteins involved in cell-cell and/or cell-matrix contacts are N-Cadherin, E-cadherin (CDH1), CD44, vimentin or fibronectin (Selgas et al., 2006, Aroeira et al., 2007).

At least one of the markers analyzed according to the invention is a growth factor promoting angiogenesis, wherein the growth factor is vascular endothelial growth factor (VEGF). Selgas et al., 2006, and Aroeira et al., 2004 teach a role for VEGF in EMT. Alternative or additional markers from the group of growth factors are TGF-β, FGF-1, FGF-2 (Aroeira et al., 2007).

Another marker analyzed according to the invention is the BMP antagonist gremlin 1 (also designated DAN family BMP antagonist or GREM). Lee et al., 2007 (Investigative Ophthalmology & Visual Science September 2007, Vol.48, 4291-4299) teach that gremlin 1 is involved in EMT in proliferative vitreoretinopathy. Preferably, the BMP antagonist GREM is determined in the supernatant of cells potentially undergoing EMT, in particular, cells from the effluent of PD patients. The inventors showed that the expression of GREM in effluent is not significantly associated with EMT, so, analysis of effluent, does not have to include analysis of the BMP antagonist GREM.

Optional further markers include Tissue Factor Pathway Inhibitor (TFI2), Thrombomodulin (THBD), Aquaporin 1 (AQP1) and/or Kinase Insert Domain Receptor (KDR).

According to a further embodiment of the invention, an increased amount of the markers discussed above indicates an epithelial-to-mesenchymal transition (EMT) of the peritoneum. The amount of markers can be compared to a control sample, e.g., a sample taken from a healthy subject, e.g., a human not undergoing PD, preferably, a pool of such control samples comprising samples from at least 2, at least 3, at least 4, at least 5, at least 10 or at least 20 healthy subjects. Alternatively, the amount of marker in the sample that it to be analyzed can be determined using standards, and the results compared with results obtained from control samples or a pool thereof.

It is also possible to compare the amount of markers of the same subject at two or more time points, e.g., before treatment (e.g., before PD, if status of the peritoneum is to be analyzed), after treatment, e.g., with PD for specific times, e.g., after at least 1 month, after at least 6 months, after at least 1 year, after at least 2 years, at least 3 years, at least 4 years, at least 5 years, at least 6 years, at least 7 years, at least 8 years, at least 9 years, at least 10 years, at least 11 years, at least 12 years, at least 13 years, at least 14 years, at least 15 years or at least 20 years. As EMT can be reversible, an analysis can also be carried out after a change in treatment regimen or a change in the PD fluid used.

Throughout the invention, the epithelial-to-mesenchymal transition is epithelial-to-mesenchymal transition of the peritoneum, wherein, according to a preferred embodiment of the invention, the sample is derived from a peritoneal dialysis patient, e.g., a PD patient having chronic kidney disease. The inventors have surprisingly found that the set of markers of the invention shows a close association with function of the peritoneum, in particular, the efficacy of filtration by the peritoneum.

The PD may be, e.g., (CAPD (continuous ambulatory peritoneal dialysis), or CCPD (continuous cyclic PD), IPD (intermittent PD), or NIPD (nightly intermittent PD).

It may also be of interest to analyze EMT involved in embryogenesis or wound healing. Diagnosis of EMT is of clinical relevance, in addition to PD, in cancer progression and metastasis, in particular, in abdominal cancer progression and peritoneal metastasis, or in degenerative fibrotic disorders in different organs, including the lung (as disclosed in WO 2014/139885 A2), or in post-surgical peritoneal adhesion formation. In general, EMT can be a marker for peritoneal diseases.

The sample may be derived from any biological sample potentially undergoing EMT. For example, it may be a sample derived from an organ or tissue undergoing a degenerative fibrotic disorder. It may be a patient's body fluid, e.g., peritoneal fluid, blood, serum or plasma of a patient. If the sample is from a PD patient, the sample is preferably selected from peritoneal effluent (i.e., used peritoneal dialysate), peritoneal fluid, serum, plasma, or peritoneal tissue, or it may be derived therefrom, e.g., supernatant of cultivated cells from peritoneal effluent. The inventors have shown that excellent results can be obtained on the basis of peritoneal effluent.

The sample may also be selected from culture medium or cell lysate from a cell or tissue culture useful as a model for EMT, e.g., a model for peritoneal dialysis. For example, ARPE-19 cells were shown to be a useful model for EMT (Lee et al., 2007). An alternative model would be, e.g., a mouse model of PD.

The present invention also provides use of the kit for diagnosis of epithelial-to-mesenchymal transition (EMT) of the peritoneum of a peritoneal dialysis patient. The status of the EMT, in particular, the status of the peritoneum can be determined using the kit and the method of the invention, wherein it is possible to differentiate between early and advanced phases.

The present invention also provides a method for predicting the progression of epithelial-to-mesenchymal transition of the peritoneum of a patient, comprising analyzing the status of the peritoneum of the patient with a kit and/or the method of the invention. As disclosed herein, alternatively to peritoneal tissue, the tissue may also be a cancer, thus allowing for prediction of progression and/or metastasis of the cancer.

The present invention also provides a method for optimizing the therapy of a peritoneal dialysis patient, comprising analyzing the status of the peritoneum of the patient with a kit and/or method of the invention, wherein the therapy is adapted to the status of the patient's peritoneum. The therapy may be adapted in a way that allows for the longest possible use of peritoneal dialysis, and/or in a way that optimizes efficiency of peritoneal dialysis.

The present invention also provides a method for testing a PD solution, comprising
a) contacting a peritoneum or a cell or tissue culture serving as a model of a peritoneum with the PD solution, and
b) analyzing the status of the peritoneum or the cell or tissue culture with a kit and/or method of any of the invention.

The method for testing a PD solution may be carried out in vivo or in vitro. The inventors showed that Aquaporin 1 (AQP1) expression correlates with use of bioincompatible PD fluids, so analysis of this marker is preferably included for testing PD solutions.

The invention is further illustrated by the following figures, examples and exemplary embodiments, which are not intended to limit the invention.

### Figures

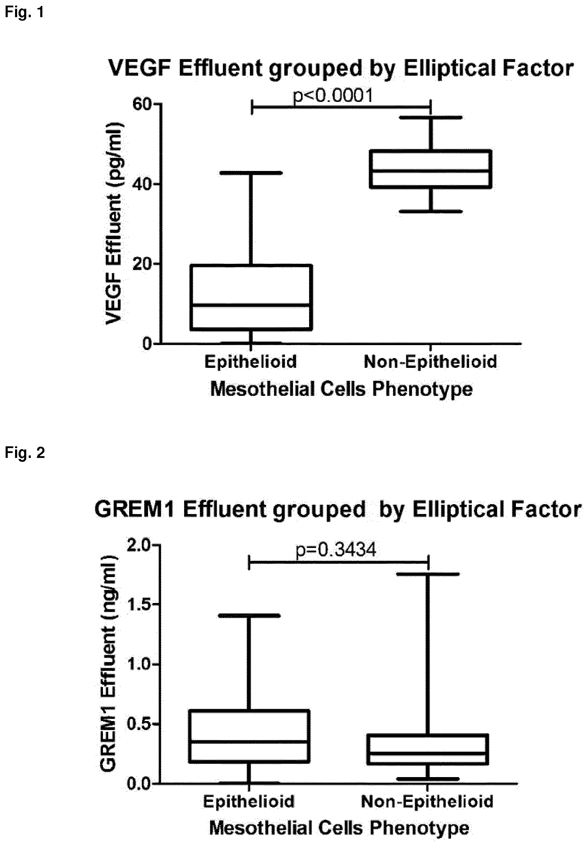

- **Fig. 1**: shows expression of VEGF in effluent from patients that have been categorized into E (Epithelioid) and NE (Non-Epithelioid), wherein the protein was quantified with antibodies. Categorization was based on Elliptical Factor. Two photographs were taken of each cell culture. Ten cells were randomly chosen and were measured the major and minor axis of cell and calculated a ratio. The mean of each cultured was calculated and if this mean was equal or higher than 2 (the cell was double of long that wide) the culture was classificated as non-epitelioid, and, otherwise, as epitelioid. Epithelioid=12.73±12.81 pg/ml (N=20); Non-Epithelioid=43.70±6.181 pg/ml (N=20)
- **Fig. 2**: shows expression of GREM1 in effluent from patients that have been categorized into E (Epithelioid) and NE (Non-Epithelioid) as above, wherein the protein was quantified with antibodies. Epithelioid=0.4405±0.3598 ng/ml (N=20); Non-Epithelioid=0.3555±0.3756 ng/ml (N=20)
- **Fig. 3**: shows expression of Thrombospondin 1 in effluent from patients that have been categorized into E (Epithelioid) and NE (Non-Epithelioid) as above, wherein the protein was quantified with antibodies. Epithelioid=5.791±7.669 ng/ml (N=20); Non-Epithelioid=66.62±42.18 ng/ml (N=20)
- **Fig. 4**: shows expression of Thrombospondin 1 in effluent from patients that have been categorized according to Mass Transfer Coefficient of creatinine (Cr-MTC), i.e., a parameter to determinate the transport across the peritoneal membrane, wherein the protein was quantified with antibodies. Cr-MTC<11=19.19±26.11 ng/ml (N=26); Cr-MTC>11=67.81±50.66 ng/ml (N=14)

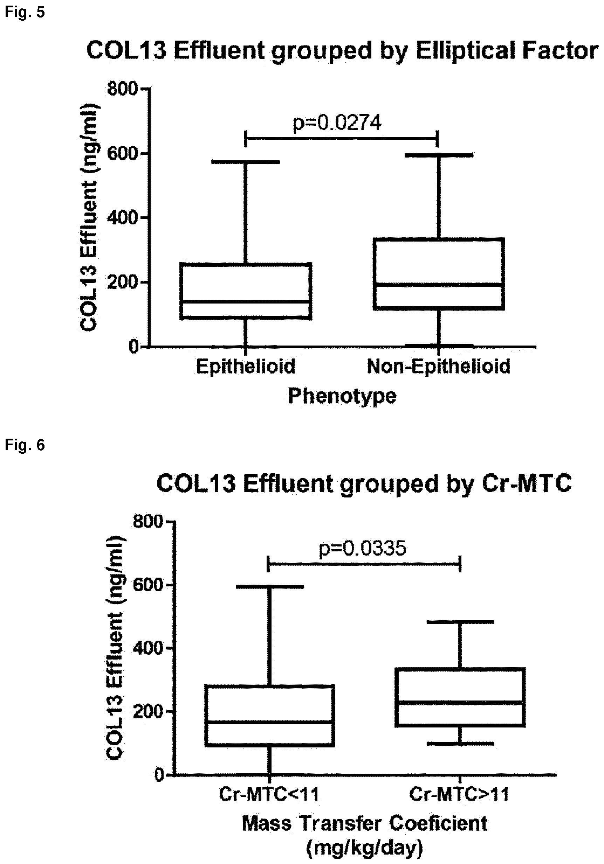

- **Fig. 5**: shows expression of COL 13 in effluent from patients that have been categorized according to Elliptical Factor, wherein the protein was quantified with antibodies. Epithelioid=166.4±120.1 ng/ml, N=33; Non-Epithelioid=228.0±132.4 ng/ml, N=43
- **Fig. 6**: shows expression of COL 13 in effluent from patients that have been categorized according to Mass Transfer Coefficient of creatinine (Cr-MTC), wherein the protein was quantified with antibodies. Cr-MTC<11=189.8±132.8 ng/ml, N=51; Cr-MTC>11=243.8±108.6 ng/ml, N=23

### Examples

### Example 1

Differential gene expression studies were performed in cultured mesothelial cells derived from effluents of PD patients. 9 samples with Epithelioid and 8 samples with Non-Epithelioid phenotypes derived from PD patients were compared with a control pool (mesothelial cells derived from four different healthy donors' omentum). Different groups of induced or repressed genes were obtained and 40 genes were validated by RT-qPCR. Results for subset of 15 genes are shown in Table 1. Furthermore, results were grouped according to use of biocompatible (e.g. BicaVera) and less biocompatible ("bioincompatible") PD fluids (e.g. Stay-safe), as shown in Table II. Table III shows that the use of bioincompatible PD fluids leads to a higher rate of EMT than use of biocompatible PD fluids. The results in Tables I and II show the high functional significance of the markers of the invention.

mRNA of CDH13, COL6A3, COL13A1, KRT34, MMP1, THBS1 (also designated TPS1), VEGFA and CDH1 showed a statistic difference between Epithelioid and Non-Epithelioid phenotypes and between the characteristic bio-compatible and bio-incompatible of PD fluids. CD44, TFI2, KDR and THBD showed a significant difference between Epithelioid and Non-Epithelioid phenotypes, and AQP1 differed between bio-compatible and bio-incompatible PD fluids.

**Table I:**

| **GEN** | **Epithelioid** | | | **Non-Epithelioid** | | | **p-value** |
|---|---|---|---|---|---|---|---|
| | **N** | **Mean** | **SD** | **N** | **Mean** | **SD** | |
| **CD44** | 23 | 2.88 | 2.94 | 28 | 4.87 | 3.34 | 0.001 |
| **CDH13** | 23 | 15.07 | 13.96 | 28 | 26.16 | 23.03 | 0.049 |
| **COL6A3** | 23 | 8.80 | 10.07 | 28 | 18.03 | 20.91 | 0.020 |
| **COL13A1** | 23 | 1.39 | 1.34 | 28 | 3.16 | 3.54 | 0.016 |
| **GREM1** | 23 | 4.03 | 4.14 | 28 | 7.61 | 11.97 | 0.798 |
| **IL33** | 23 | 2.42 | 2.26 | 28 | 4.90 | 5.78 | 0.108 |
| **KRT34** | 23 | 7.59 | 8.23 | 28 | 27.21 | 33.15 | 0.012 |
| **MMP1** | 23 | 1.37 | 1.25 | 28 | 6.73 | 9.83 | 0.002 |
| **MMP2** | 23 | 3.97 | 3.90 | 28 | 4.64 | 3.27 | 0.116 |
| **TFPI2** | 23 | 5.80 | 5.35 | 28 | 10.57 | 9.91 | 0.016 |
| **THBS1** | 23 | 7.36 | 8.54 | 28 | 19.41 | 24.98 | 0.022 |
| **VEGFA** | 23 | 1.84 | 1.32 | 28 | 3.32 | 3.29 | 0.088 |
| **CDH1** | 23 | 0.61 | 0.46 | 28 | 0.25 | 0.25 | 0.001 |
| **KDR** | 23 | 0.87 | 0.55 | 28 | 0.63 | 0.52 | 0.041 |
| **THBD** | 23 | 0.46 | 0.53 | 28 | 0.19 | 0.12 | 0.047 |

**Table II:**

| **GEN** | **Biocompatible** | | | **Bioincompatible** | | | **p-value** |
|---|---|---|---|---|---|---|---|
| | **N** | **Mean** | **SD** | **N** | **Mean** | **SD** | |
| **CD44** | 28 | 3.53 | 2.94 | 23 | 4.51 | 3.66 | 0.135 |
| **CDH13** | 28 | 12.39 | 8.67 | 23 | 31.84 | 24.64 | 0.003 |
| **COL6A3** | 28 | 8.06 | 8.19 | 23 | 20.93 | 22.57 | 0.006 |
| **COL13A1** | 28 | 1.37 | 1.27 | 23 | 3.57 | 3.77 | 0.006 |
| **GREM1** | 28 | 5.45 | 9.19 | 23 | 6.66 | 9.80 | 0.977 |
| **IL33** | 28 | 3.70 | 5.34 | 23 | 3.88 | 3.82 | 0.140 |
| **KRT34** | 28 | 8.73 | 10.64 | 23 | 30.08 | 35.17 | 0.010 |
| **MMP1** | 28 | 4.27 | 9.49 | 23 | 4.36 | 5.12 | 0.066 |
| **MMP2** | 28 | 3.61 | 3.02 | 23 | 5.23 | 3.98 | 0.096 |
| **TFPI2** | 28 | 8.42 | 6.66 | 23 | 8.42 | 10.38 | 0.691 |
| **THBS1** | 28 | 5.78 | 5.31 | 23 | 23.96 | 26.41 | 0.000 |
| **VEGFA** | 28 | 1.90 | 1.85 | 23 | 3.57 | 3.24 | 0.007 |
| **CDH1** | 28 | 0.38 | 0.43 | 23 | 0.46 | 0.35 | 0.061 |
| **KDR** | 28 | 0.66 | 0.48 | 23 | 0.83 | 0.60 | 0.325 |
| **THBD** | 28 | 0.37 | 0.50 | 23 | 0.24 | 0.18 | 1.000 |

**Table III:**

| | **Bioincompatible** | **Biocompatible** | **Total** |
|---|---|---|---|
| **Non-Epithelioid** | 30 | 16 | 46 |
| **Epithelioid** | 15 | 19 | 34 |
| **Total** | 45 | 35 | 80 |

| | | | |
|---|---|---|---|
| p-value = 0.06 | | | |

### Example 2

By Enzyme-Linked ImmunoSorbent Assay (ELISA), protein was determined in supernatant obtained from cultures of mesothelial cells derived from 26 PD patient effluents, distributed like this: 26 samples taken at PD treatment start, 26 samples taken at 12 months, 20 samples taken at 18 months and 11 samples taken at 24 months (data not shown). Mesothelial cells were cultured in Earle's M199 medium supplemented with 20% of FBS and 50 U/ml penicillin, 50 µg/ml streptomycin and 2% of Biogro-2. When cells reached confluency, medium was replaced with fresh medium during 24h. Supernatant was collected and stored at -80°C until use. For analysis of supernatants, the protein in supernatant was normalized with the total protein in the cell lysate. Selected markers were analyzed with ELISA. TSP1, VEGF, MMP2, CDH13 and GREM1 showed different expression between Epithelioid and Non-Epithelioid phenotypes, with a higher amount of all of these proteins in the Non-Epithelioid group.

### Example 3

40 effluents derived from PD patients were analyzed by ELISA, and exemplary data for different markers on protein level is included herein.

VEGF in effluent showed a significant difference between Epithelioid and Non-Epithelioid phenotypes (Fig. 1). This indicates that according to progress of EMT of mesothelial cells, VEGF increases. Mass Transfer Coefficient (MTC) of creatinine (Cr) is a clinical parameter than indicates the peritoneal transport state. MTC higher than 11 is characteristic for a pathological peritoneal transport. When results for VEGF in effluent were grouped by MTC, a statistic difference was observed between patients with MTC higher or lower than 11, and VEGF was higher in MTC>11 group. So, VEGF in effluent may be used to determine the functional state of the peritoneal membrane. VEGF in effluent, when grouped by bio-incompatible (more aggressive fluid) and bio-compatible PD fluids, also showed a significant difference between these groups with more VEGF in bio-incompatible fluid.

GREM1 in effluent didn't show a significant difference between Epithelioid and Non-Epithelioid phenotypes (Fig. 2), with a higher amount of protein in Epithelioid group, but the difference was significant in supernatant, where Non-Epithelioid phenotype present the higher amount of protein. GREM1 in effluent presented statistic difference when it was classified by MTC, with the higher level of protein in MTC<11 group. GREM1 did not show a significant difference between bio-compatible and bio-incompatible PD fluids.

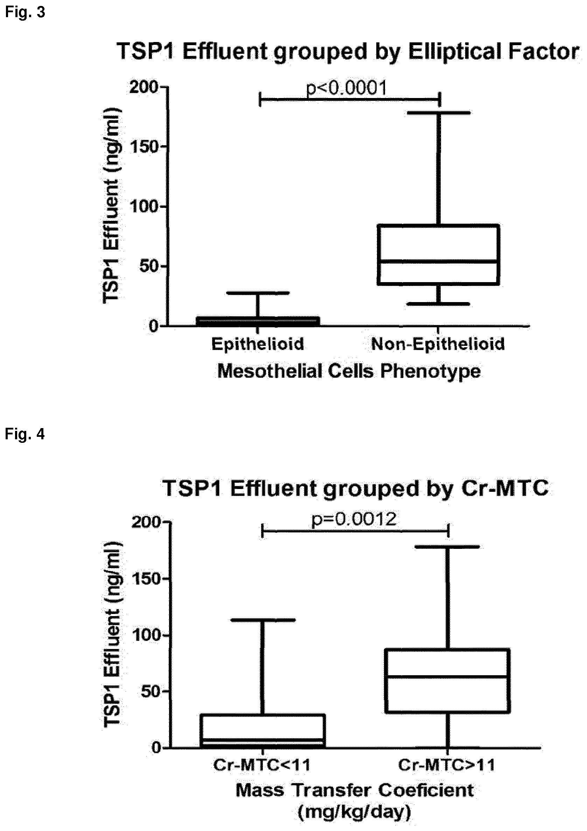

TSP1 in effluent showed a significant difference between Epithelioid and Non-Epithelioid phenotypes (Fig. 3) and between MTC<11 or MTC>11 groups (Fig. 4). So, TSP1 is related with the EMT of mesothelial cell/MMT process and the transport in peritoneal membrane. TSP1 protein in effluent didn't show differences between bio-compatible and bio-incompatible PD fluids, but the p-value (p=0.16) indicate a tendency to significance.

COL13 in effluent has been also analyzed and showed differences depending on phenotypes and creatinine transport.

## Claims

1. Use of a kit for the detection of markers in a sample for the diagnosis of epithelial-to-mesenchymal transition (EMT) of the peritoneum, the kit comprising agents for the detection of the markers cadherin 13, collagen 13, collagen 6, keratin 34, matrix metalloproteinase 1, thrombospondin 1, VEGF and Gremlin 1, wherein the sample is selected from the group comprising
a) a sample from a patient comprising peritoneal effluent, peritoneal fluid, serum, peritoneal tissue, and
b) culture medium or cell lysate from a cell or tissue culture useful as a model for peritoneal dialysis,
wherein the agents for the detection of the markers are antibodies or fragments thereof which are suitable for the detection of the markers in protein form, or nucleic acids which are suitable for the detection of the markers in nucleic acid form.

2. A method for the diagnosis of epithelial-to-mesenchymal transition (EMT) of the peritoneum, comprising detecting the absence and/or amount of markers in a sample, wherein the markers are cadherin 13, collagen 13, collagen 6, keratin 34, matrix metalloproteinase 1, thrombospondin 1, VEGF and Gremlin 1, wherein the sample is selected from the group comprising
a) a sample from a patient comprising peritoneal effluent, peritoneal fluid, serum, peritoneal tissue, and
b) culture medium or cell lysate from a cell or tissue culture useful as a model for peritoneal dialysis.

3. The method of claim 2, wherein the agents for the detection of the markers in the sample are antibodies or fragments thereof.

4. The use of claim 1 or the method of claims 2 or 3, wherein the agents for the detection of the markers in the sample are linked to a solid support, wherein the kit preferably comprises an antibody chip.

5. The use of any of claims 1 or 4 or the method of any of claims 2-4, wherein an increased amount of the markers indicates an epithelial-to-mesenchymal transition (EMT) of the peritoneum.

6. The use of any of claims 1, 4 or 5 or the method of any of claims 2-5, wherein the sample is derived from a peritoneal dialysis patient.

7. A method for predicting the progression of epithelial-to-mesenchymal transition of the peritoneum of a patient, comprising analyzing the status of the peritoneum of the patient with a kit as defined in any of claims 1 or 4-6, and/or with a method of any of claims 2-6.

8. A method for optimizing the therapy of a peritoneal dialysis patient, comprising analyzing the status of the peritoneum of the patient with a kit as defined in any of claims 1 or 4-6, and/or with a method of any of claims 2-6, wherein the therapy is adapted to the status of the patient's peritoneum.

9. A method for testing a peritoneal dialysis solution, comprising
a) contacting a peritoneum or a cell or tissue culture serving as a model of a peritoneum with the solution
b) analyzing the status of the peritoneum or the cell or tissue culture with a kit as defined in any of claims 1 or 4-6,
and/or with a method of any of claims 2-6.

## Patentansprüche

1. Verwendung eines Kits zur Detektion von Markern in einer Probe zur Diagnose der epithelial-mesenchymalen Transition (EMT) des Peritoneums, wobei das Kit Agenzien zur Detektion der Marker Cadherin 13, Collagen 13, Collagen 6, Keratin 34, Matrixmetalloproteinase 1, Thrombospondin 1, VEGF und Gremlin 1 umfasst, wobei die Probe ausgewählt ist aus der Gruppe, umfassend
a) eine Probe von einem Patienten, umfassend Peritonealeffluat, Peritonealflüssigkeit, Serum, Peritonealgewebe, und
b) Kulturmedium oder Zelllysat von einer Zell- oder Gewebskultur, die als Modell für Peritonealdialyse nützlich ist,
wobei es sich bei den Agenzien zur Detektion der Marker um Antikörper oder Fragmente davon handelt, die für die Detektion der Marker in Proteinform geeignet sind, oder um Nukleinsäuren, die für die Detektion der Marker in Nukleinsäureform geeignet sind.

2. Verfahren zur Diagnose der epithelial-mesenchymalen Transition (EMT) des Peritoneums, umfassend die Detektion der Abwesenheit und/oder der Menge von Markern in einer Probe, wobei die Marker Cadherin 13, Collagen 13, Collagen 6, Keratin 34, Matrixmetalloproteinase 1, Thrombospondin 1, VEGF und Gremlin 1 sind, wobei die Probe ausgewählt ist aus der Gruppe, umfassend
a) eine Probe von einem Patienten, umfassend Peritonealeffluat, Peritonealflüssigkeit, Serum, Peritonealgewebe, und
b) Kulturmedium oder Zelllysat von einer Zell- oder Gewebskultur, die als Modell für Peritonealdialyse nützlich ist.

3. Verfahren nach Anspruch 2, wobei die Agenzien zur Detektion der Marker in der Probe Antikörper oder Fragmente davon sind.

4. Verwendung nach Anspruch 1 oder Verfahren nach Anspruch 2 oder 3, wobei die Agenzien zur Detektion der Marker in der Probe an einen festen Träger gebunden sind, wobei das Kit vorzugsweise einen Antikörper-Chip umfasst.

5. Verwendung nach einem der Ansprüche 1 oder 4 oder Verfahren nach einem der Ansprüche 2 bis 4, wobei eine erhöhte Menge der Marker eine epithelial-mesenchymale Transition (EMT) des Peritoneums anzeigt.

6. Verwendung nach einem der Ansprüche 1, 4 oder 5 oder Verfahren nach einem der Ansprüche 2 bis 5, wobei die Probe von einem Peritonealdialysepatienten stammt.

7. Verfahren zur Vorhersage des Fortschritts der epithelial-mesenchymalen Transition des Peritoneums eines Patienten, umfassend das Analysieren des Status des Peritoneums des Patienten mit einem Kit, wie es in einem der Ansprüche 1 oder 4 bis 6 definiert ist, und/oder mit einem Verfahren nach einem der Ansprüche 2 bis 6.

8. Verfahren zur Optimierung der Behandlung eines Peritonealdialysepatienten, umfassend das Analysieren des Status des Peritoneums des Patienten mit einem Kit, wie es in einem der Ansprüche 1 oder 4 bis 6 definiert ist, und/oder mit einem Verfahren nach einem der Ansprüche 2 bis 6, wobei die Behandlung an den Status des Peritoneums des Patienten angepasst wird.

9. Verfahren zum Testen einer Peritonealdialyselösung, umfassend
a) Inkontaktbringen eines Peritoneums oder einer Zell- oder Gewebskultur, die als Modell für ein Peritoneum dient, mit der Lösung,
b) Analysieren des Status des Peritoneums oder der Zell- oder Gewebskultur mit einem Kit, wie es in einem der Ansprüche 1 oder 4 bis 6 definiert ist, und/oder mit einem Verfahren nach einem der Ansprüche 2 bis 6.

## Revendications

1. Utilisation d'un kit pour la détection de marqueurs dans un échantillon pour le diagnostic de la transition épithélio-mésenchymateuse (TEM) du péritoine, le kit comprenant des agents pour la détection des marqueurs cadhérine 13, collagène 13, collagène 6, kératine 34, métalloprotéinase matricielle 1, thrombospondine 1, VEGF et gremline 1, dans lequel l'échantillon est sélectionné dans le groupe comprenant
a) un échantillon d'un patient comprenant un effluent péritonéal, un fluide péritonéal, du sérum, un tissu péritonéal, et
b) un milieu de culture ou un lysat cellulaire d'une culture cellulaire ou tissulaire utile en tant que modèle pour la dialyse péritonéale,
dans lequel les agents pour la détection de marqueurs sont des anticorps ou des fragments de ceux-ci qui sont adaptés à la détection des marqueurs sous la forme de protéines, ou des acides nucléiques qui sont adaptés à la détection des marqueurs sous la forme d'acides nucléiques.

2. Procédé de diagnostic d'une transition épithélio-mésenchymateuse (TEM) du péritoine, comprenant la détection de l'absence et/ou de la quantité de marqueurs dans un échantillon, dans lequel les marqueurs sont la cadhérine 13, le collagène 13, le collagène 6, la kératine 34, la métalloprotéinase matricielle 1, la thrombospondine 1, le VEGF et la gremline 1, dans lequel l'échantillon est sélectionné dans le groupe comprenant
a) un échantillon d'un patient comprenant un effluent péritonéal, un fluide péritonéal, du sérum, un tissu péritonéal, et
b) un milieu de culture ou un lysat cellulaire d'une culture cellulaire ou tissulaire utile en tant que modèle pour la dialyse péritonéale.

3. Procédé selon la revendication 2, dans lequel les agents pour la détection des marqueurs dans l'échantillon sont des anticorps ou des fragments de ceux-ci.

4. Utilisation selon la revendication 1 ou le procédé selon les revendications 2 ou 3, dans lequel les agents pour la détection des marqueurs dans l'échantillon sont liés à un support solide, dans lequel le kit comprend de préférence une puce à anticorps.

5. Utilisation selon l'une quelconque des revendications 1 ou 4 ou le procédé selon les revendications 2 à 4, dans lequel une quantité accrue des marqueurs indique une transition épithélio-mésenchymateuse (TEM) du péritoine.

6. Utilisation selon l'une quelconque des revendications 1, 4 ou 5 ou le procédé selon l'une quelconque des revendications 2 à 5, dans lequel l'échantillon est dérivé d'un patient sous dialyse péritonéale.

7. Procédé de prédiction de la progression d'une transition épithélio-mésenchymateuse du péritoine d'un patient, comprenant l'analyse du statut du péritoine du patient avec un kit selon l'une quelconque des revendications 1 ou 4 à 6, et/ou avec un procédé selon l'une quelconque des revendications 2 à 6.

8. Procédé d'optimisation de la thérapie d'un patient sous dialyse péritonéale, comprenant l'analyse du statut du péritoine du patient avec un kit selon l'une quelconque des revendications 1 ou 4 à 6, et/ou avec un procédé selon l'une quelconque des revendications 2 à 6, dans lequel la thérapie est adaptée au statut du péritoine du patient.

9. Procédé de test d'une solution de dialyse péritonéale, comprenant
a) la mise en contact d'un péritoine ou d'une culture cellulaire ou tissulaire servant de modèle de péritoine avec la solution
b) l'analyse du statut du péritoine ou de la culture cellulaire ou tissulaire avec un kit selon l'une quelconque des revendications 1 ou 4 à 6, et/ou avec un procédé selon l'une quelconque des revendications 2 à 6.
